# FASCICULE DE BREVET EUROPEEN

(11) **EP 4 000 577 B1**
(45) Date de publication et mention de la délivrance du brevet: **19.02.2025**
(21) Numéro de dépôt: 21185382.5
(22) Date de dépôt: 13.07.2021
(51) Int. Cl.: A61H 1/00, A61F 6/00, A61H 39/04, A61F 5/28, A61F 2/00

(54) **DISPOSITIF DE MAINTIENT EXTERNE DE L'URÈTRE FÉMININ À L'EFFORT**
VORRICHTUNG ZUR EXTERNEN STÜTZUNG DER WEIBLICHEN HARNRÖHRE BEI ANSTRENGUNG
DEVICE FOR EXTERNAL SUPPORT OF THE FEMALE URETHRA UNDER FORCE

(30) Priorité: 17.11.2020 FR 2011794; 23.06.2021 FR 2106711
(43) Date de publication de la demande: 25.05.2022
(73) Titulaire: Eurl Cornier, 75116 Paris (FR)
(72) Inventeur: CORNIER, Edgard, 75116 Paris (FR)
(74) Mandataire: Herrburger, Pierre

(56) Documents cités:
- EP-A1- 2 158 882
- EP-B1- 2 158 882
- JP-A- 2014 151 022
- US-B1- 8 672 910

## Description

### DOMAINE DE L'INVENTION

La présente invention a pour objet un dispositif de maintien externe de l'urètre féminin à l'effort.

### ETAT DE LA TECHNIQUE

Selon des études scientifiques, l'incontinence *urinaire* affecte environ 20% des femmes jeunes et 77% à l'age du relâchement tissulaire ( Urinary Incontinence in Women: A Review.

Lukacz ES, Santiago-Lastra Y, Albo ME, Brubaker L.JAMA. 2017 Oct 24;318(16):1592-1604.)

L'arsenal thérapeutique est pauvre et la plupart des femmes affectées sont réduites au port de couches absorbantes.

La rééducation musculaire à elle seule est peu efficace dans la durée, ou contraignante. Il faut lui adjoindre différentes méthodes, qui supposent un investissement personnel difficile à assumer sur le long terme. L'arrêt de l'entrainement s'accompagne de récidives.

Kegel E xercises, Biofeedback, Electrostimulation, and Peripheral Neuromodulation Improve Clinical Symptoms of Fecal Incontinence and Affect Specific Physiological Targets: An Randomized Controlled Trial. Mundet L, Rofes L, Ortega O, Cabib C, Clavé P.J Neurogastroenterol Motil. 2020 Oct 28.

La mise en place de pessaires intra vaginaux est inconfortable, peu suivie, peu efficaces sur les fuites urinaires. Pessary use in stress urinary incontinence: a review of advantages, complications, patient satisfaction, and quality of life.

Al-Shaikh G, Syed S, Osman S, Bogis A, Al-Badr A.Int J Womens Health. 2018

Les dispositifs de soutien de l'ensemble du périnée selon l'arrière-plan technologique, on connaît les documents suivants selon : EP 2 158 882, US 2015/034074, JP 2014 1510-20. Ces documents antérieurs proposent des dispositifs placés sous tout le périnée et maintenus par des bretelles. Leur but est d'empêcher les descentes des prolapsus, et les refoulant largement, à l'aide d'un dispositif convexe ou concave, vers l'intérieur de l'orifice vaginal.

Ces dispositifs plus ou moins larges prennent appui à l'extérieur de la vulve, sur l'appui osseux de la branche inférieure de l'os pubien jusqu'à l'Ischion. Ils comportent parfois une gouttière en avant pour permettre aux urines de s'échapper. Aucun de ces dispositifs ne revendique le traitement des fuites urinaires de la femme.

Ces dispositifs soulèvent, l'ensemble des organes du petit bassin très au-delà de la zone postérieure du périnée, et soulèvent la totalité des tissus pelviens. Ils sont destinés à maitriser les grandes descentes d'organe. Ils répondent aux exigences du postulat communément admis par les professionnels.

De façon plus détaillée, le postulat ou 1 'hypothèse habituelle se réfèrent aux documents d'anatomie enseignes (Kamina Anatomie Clinique Tome 4 - 2ème Ed. - 4ème tirage -Maloine 2012 - 383p). Selon ces documents, les viscères sont solidement soutenus et suspendus (p.250).

Cette approche reprend le postulat de l'existence d'un plancher pelvien de soutien. Les dispositifs connus, convexes, recouvrent la totalité du périnée. Pour soutenir les forces de poussée qui se dirigent directement vers le périnée.

Selon l'état de la technique cité ci-dessus, les dispositifs connus sont de forme convexe, souple, large et longue, recouvrant toute la surface périnéale.

Le document US 8 672 910, décrit un dispositif masculin est en forme de bossage à sommet plat, complet, rigide, large et long recouvrant toute la surface périnéale.

### BUT DE L'INVENTION

La présente invention a pour but de développer un dispositif de soutien remédiant aux inconvénients des solutions connues et de permettre la plicature de l'urètre lors d'un effort abdominal.

### EXPOSE ET AVANTAGES DE L'INVENTION

A cet effet, l'invention a pour objet un dispositif de soutien directionnel externe de l'urètre féminin à l'effort, selon le jeu de revendications.

Le dispositif de soutien directionnel selon l'invention permet aussi de réorienter les forces de pression abdominale, lors de l'effort abdominal, grâce à la mise en place postérieure de ce contre-appui réalisé par la plaquette rigide et courte gardant sa forme plate, placée en avant du coccyx, ne recouvrant pas l'ensemble de la surface périnéale et restant à distance de l'orifice urinaire. Il permettra la plicature de 1 'urètre. Cette plicature suffit à empêcher les fuites urinaires.

Ce concept novateur n'a pas été accepté sans les tests qui ont apporté la preuve de son efficacité. Ainsi une personnalité scientifique a pu évaluer son intérêt novateur.

Grâce au dispositif de maintien selon l'invention, les pressions produisent des mouvements quasi statiques de plicature des organes entre eux. (reference A2499EC European Journal of Obstetrics & Gynecology and Reproductive Biology 234 (2019) - From tissue viscoelasticity to vectorial perineology - Edgard Cornier1,2, *, et al.). La Plicature des organes créent des zones appelées « fonctionnelles ». Ce sont ces zones qui font obstacles aux fuites. L'invention consiste à corriger ces renvois de forces afin de recréer la plicature fonctionnelle des organes du petit bassin. Un appui postérieur permettra avec la redirection de ces vecteurs de force, la flexion de l'urètre féminin, sa clôture, et la guérison des incontinences urinaires à l' effort. Le reste du pelvis, au centre, et latéralement (les grandes lèvres et les tissus adjacents) ne doivent pas être maintenus et ils doivent rester libre de leurs mouvements.

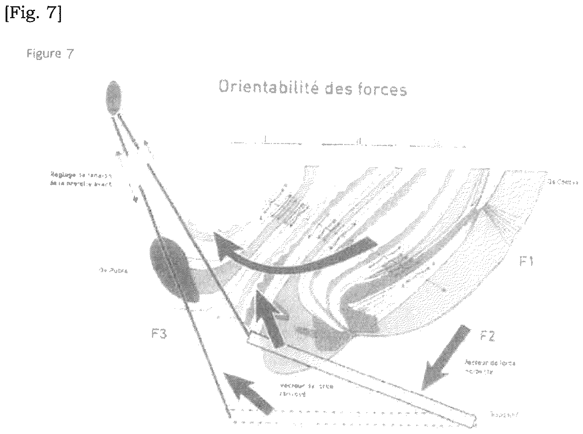

En résumé le dispositif selon l'invention permet le renvoi des forces qui engendre la plicature et l 'empilement des tissus ce qui réalise une occlusion physiologique de l'urètre empêchant la fuite urinaire (Fig. 7).

Suivant une caractéristique, la ceinture a un renforcement postérieur portant le point de fixation arrière.

Suivant une autre caractéristique, la ceinture est combinée à une culotte et notamment un coussin avec une plaquette rigide et courte, gardant sa forme plate, est intégrée dans la partie intermédiaire de la culotte reliant la partie avant et la partie arrière.

La culotte a une partie arrière renforcée au niveau de la ceinture portant le point de fixation arrière et une partie arrière rigide reliée au renforcement de la partie interfessiaire de la culotte.

Suivant une autre caractéristique avantageuse, la culotte a une partie avant munie d'une double paroi formant un canal de passage pour chacune des lanières avant pour rejoindre les deux points de fixation réglables sur le côté antérieur.

De façon avantageuse, les points de fixation réglables antérieurs sont constitués chacun par un double anneau surmontant l'orifice de sortie de la lanière correspondante. Cette réalisation permet une fixation réglable facile et simple à mettre en œuvre.

De façon avantageuse, le point de fixation réglable arrière comprend une boucle de passage fixée au renforcement postérieur et la lanière comporte une extrémité dentée d'enclipsage permettant d'accrocher la lanière à l'une des dents de la boucle pour réaliser un réglage fixe.

Ce réglage une fois effectué reste maintenu alors que le réglage au niveau des points de fixation avant reste réglable et peut être resserré ou desserré à tout moment en fonction de nécessité ou de la sensation de confort.

Le dispositif selon l'invention est orientable grâce à l'ajustement de la ceinture de tension avant. En fonction de l'intensité des efforts abdominaux, le renvoi des forces de pression peut être redirigé plus ou moins haut vers l'appui osseux de pubis. Le dispositif bloqué en arrière par l'appui osseux du coccyx, peut ainsi être ajusté et adapté au cours de la journée. La tension réglable des bretelles avant le permet. En effet le dispositif est étroit, et donc mobile entre les autres reliefs osseux latéraux des branches du pubis et de l'Ischion.

De façon particulièrement avantageuse, le coussin en forme de plaquette rigide et courte, gardant sa forme plate, est intégrée dans la partie intermédiaire de la culotte, l'avant du coussin étant muni des deux lanières passant dans les deux canaux de la double paroi avant pour rejoindre les deux points de fixation avant.

Ce mode de réalisation est particulièrement avantageux car pratique et confortable.

En longueur, il ne dépasse pas les deux tiers de la longueur du périnée. Il laisse libre le tiers antérieur du périnée.

Ce dispositif est maintenu en place par différents moyens possibles tes qu'une culotte ou une ceinture abdominale.

Les dimensions moyennes du coussin en forme de plaquette rigide et courte, gardant sa forme plate, sont de l'ordre de 5 cm à 11 cm et largeur de 2 centimètres.

Une enveloppe souple formée par une protection biocompatible entoure la partie rigide, constituée par la plaquette rigide et courte, gardant sa forme plate,

Selon une forme de réalisation la plaquette rigide porte un dispositif ou un anneau à chacune de ses extrémités permettant de le relier à la lanière postérieure et aux deux lanières antérieures. Ces lanières sont reliées à une ceinture simple ou combinée/intégrée à une culotte.

La ceinture ou la culotte comportent dans leur partie arrière un insert ou un matériau en forme de plaquette rigide et courte gardant sa forme plate.

En résumé, la ceinture formant un bandeau ou la culotte se placent sans douleur au-dessus du relief fessier au niveau des ailes iliaques. Le bandeau ou la culotte sont renforcés dans leur tiers postérieur par un insert qui permet au bandeau ou à la ceinture de la culotte de ne pas fléchir à la tension.

Ce moyen permet d'obtenir un maintien confortable à l'ensemble.

### BREVE PRESENTATION DES DESSINS

La présente invention sera décrite de manière détaillée à l'aide de mode de réalisation de dispositifs de soutien selon l'invention représentés dans les dessins annexés dans lesquels :
[Fig. 1] vue en plan schématique du dispositif selon l'invention en forme de coussin muni d'une plaquette rigide et courte gardant sa forme plate
[Fig. 2A] vue de face de l'ensemble du dispositif de coussin mis en place selon un premier mode de réalisation,
[Fig. 2B] vue arrière
[Fig. 3] vue schématique du coussin rectangulaire et de la ceinture au niveau du point de fixation arrière
[Fig. 4A] vue de détail d'un mode de réalisation de la fixation arrière
[Fig. 4B] vue de détail de deux points de fixation avant selon un mode de réalisation de l'invention
[Fig. 5] vue en perspective d'un premier mode de réalisation d'une culotte
[Fig. 6A] vue de face d'un autre mode de réalisation d'une culotte avec un coussin intégré
[Fig. 6B] vue arrière de la culotte de la figure 6A
[Fig. 6C] vue de la double paroi avant
[Fig. 7] dessin anatomique montrant l'orientabilité du dispositif ajustable

### DESCRIPTION DE MODES DE REALISATION

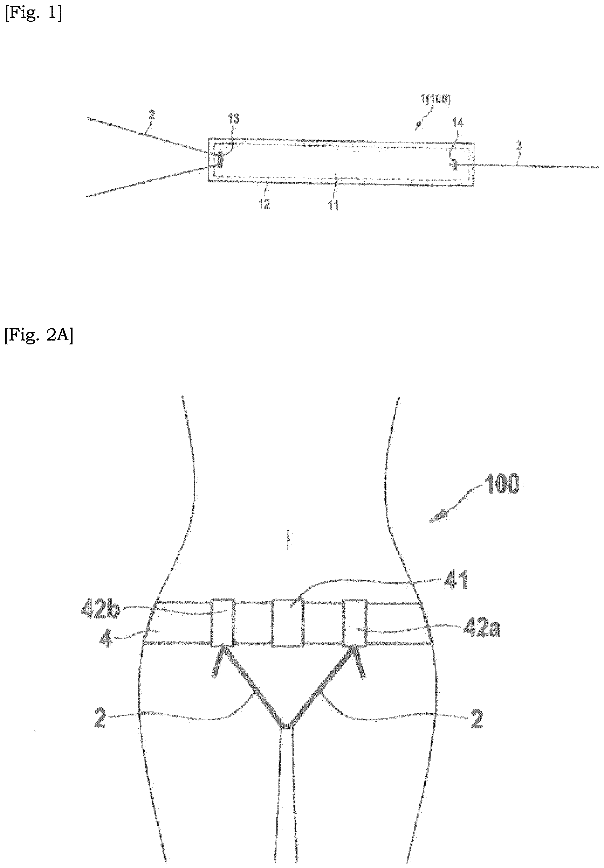

La vue de dessus schématique de la figure 1 montre un coussin 1 selon l'invention composé d'une plaquette 11 étroite, rigide et courte gardant sa forme plate, logée dans une enveloppe souple 12. La plaquette 11 est munie d'une attache avant 13 et d'une attache arrière 14. L'attache arrière 14 reçoit une lanière postérieure 2 et l'attache avant reçoit deux lanières avant 3.

La plaquette 11 étroite, rigide et courte, gardant sa forme plate a une longueur de l'ordre de 5 à 11cm et une largeur de l'ordre de 2cm. L'enveloppe souple 12 qui habille la plaquette est une enveloppe libre ou une enveloppe fixée ou intégrée à la plaquette 11.

Selon un mode de réalisation, le coussin 1 est réalisé par bi-injection en deux matières plastiques, une matière rigide gardant sa forme plate formant la plaquette 11 et une matière souple biocompatible formant l'enveloppe 12.

Le dispositif de soutien orientable directionnel externe 100 selon l'invention se compose du coussin 1 tel que représenté à la figure 1 et d'une ceinture 4 comme celle présentée aux figures 2A, 2B, 9 et 10.

La ceinture 4 est une ceinture large, élastique, munie le cas échéant d'une fermeture 41 permettant de régler la ceinture. A l'avant la ceinture 4 est munie de deux points de fixation 42a, 42b et à l'arrière d'un point de fixation arrière 43 fixé à un renfort arrière 44.

La figure 2A montre les deux lanières antérieures 2, reliées respectivement à l'un et l'autre des points de fixation 42a, 42b en divergeant en forme de V.

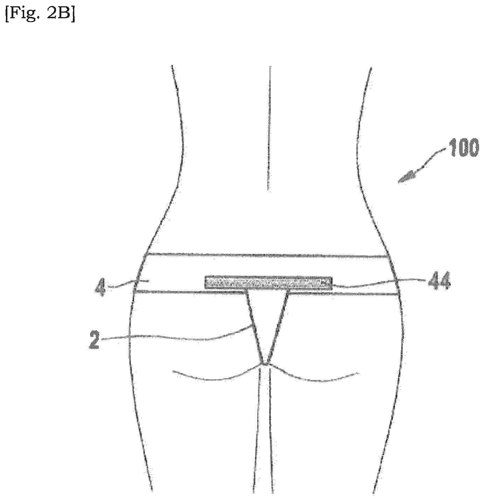

La figure 2B montre la lanière postérieure 3, reliée au point de fixation arrière 43. Les lanières avant sont de préférence élastiques et remontent vers la ceinture en s'écartant le long des plis de l'aine pour passer à travers la ceinture 4. Elles permettent le réglage de la tension à tout moment pendant le port du dispositif de soutien 100.

La ceinture 4 se place sans douleur au-dessus du relief fessier au niveau des ailes iliaque. Le bandeau formé par la ceinture 4 est renforcé dans son tiers postérieur par un renfort 44 qui permet à la ceinture de ne pas fléchir à la tension pour un maintien confortable de l'ensemble du dispositif de soutien 100.

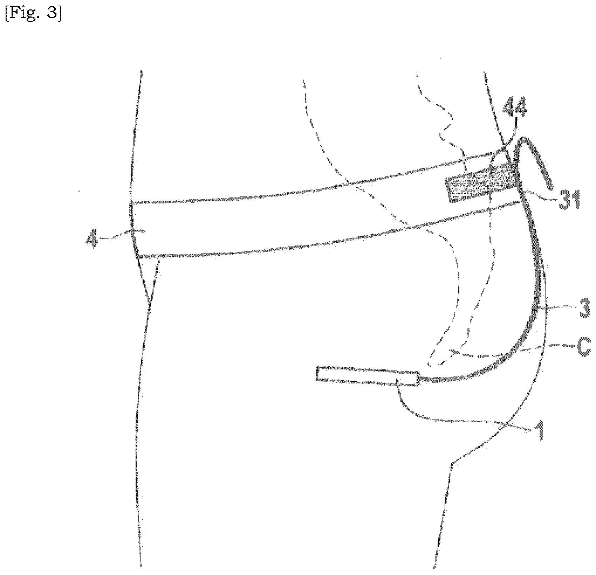

Le schéma anatomique de la figure 3 montre le positionnement de la ceinture 4 avec son renfort arrière 44 et le point de fixation arrière représenté schématiquement. Le coussin 1 est installé en avant du coccyx C.

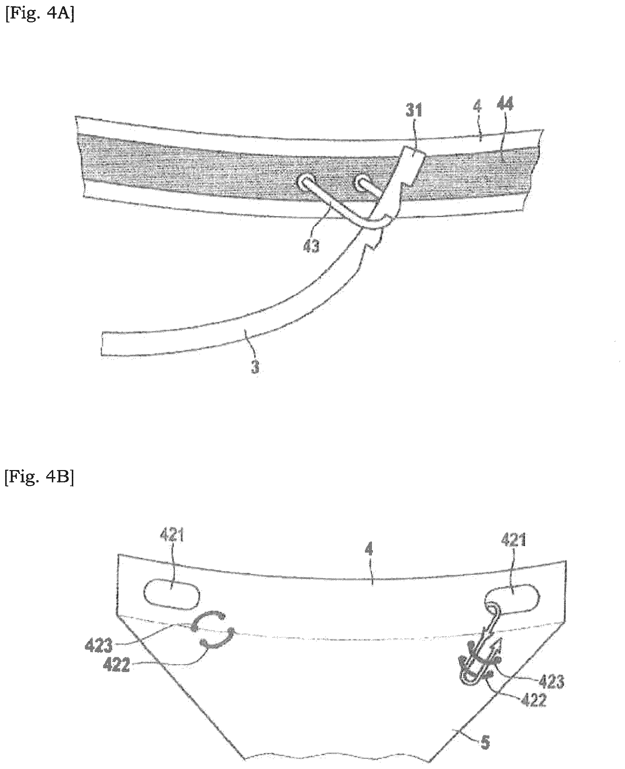

La figure 4A montre un mode de réalisation du point de fixation arrière 43 et de la lanière postérieure 3, munie de dents formant une sorte de crémaillère permettant l'accrochage de manière réglable dans la boucle constituée par le point de fixation arrière 43 relié au renfort 44 de la ceinture 4. Le réglage arrière se fait une fois la ceinture 4 mise en place. Le dispositif de réglage est constitué par la combinaison de l'anneau 43 du renfort 44 et de la partie dentée à l'extrémité de la lanière postérieure 3. Ce réglage une fois effectué reste maintenu fixe. Ainsi l'extrémité arrière du coussin 1 est mis en place définitivement contre le coccyx C.

La figure 4B montre un mode de réalisation des deux points de fixation avant 42a, 42b de la ceinture 4 représentée ici intégrée à une culotte 5.

La ceinture comporte deux orifices 421 de passage des lanières antérieures 2 à droite et à gauche et au niveau de ces orifices, deux anneaux couplés 422, 423 permettant le passage en S de la lanière antérieure 2 à travers le premier anneau 422 puis autour du second anneau 423 de façon que les deux boucles de la lanière 2 soient bloquées entre les deux anneaux 422, 423 par la traction de la lanière 2 reliée à l'extrémité avant du coussin 1.

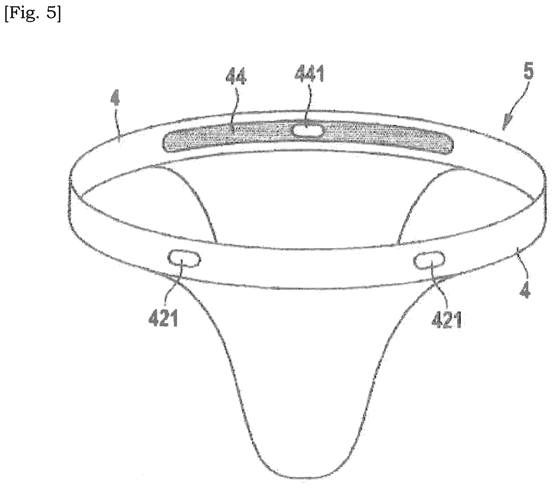

La figure 5 est une vue en perspective très schématique d'une ceinture 4 intégrée à une culotte 5. La ceinture 4 a la structure décrit ci-dessus avec deux orifices 421 à l'avant pour le passage des lanières antérieures 2 et à l'arrière un renfort 44 muni d'une ouverture 441 pour le passage de la lanière arrière 3. Cette ouverture 441 peut être réalisée dans le renfort 4ou dans la partie arrière de la culotte en-dessous de la ceinture 4.

Le point de fixation arrière 43 extérieur à la ceinture 4 n'est pas visible à la figure 5.

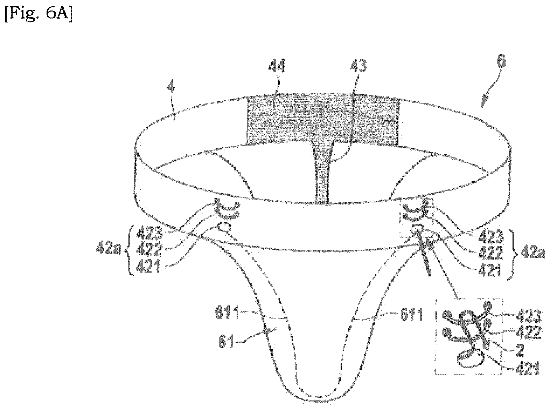

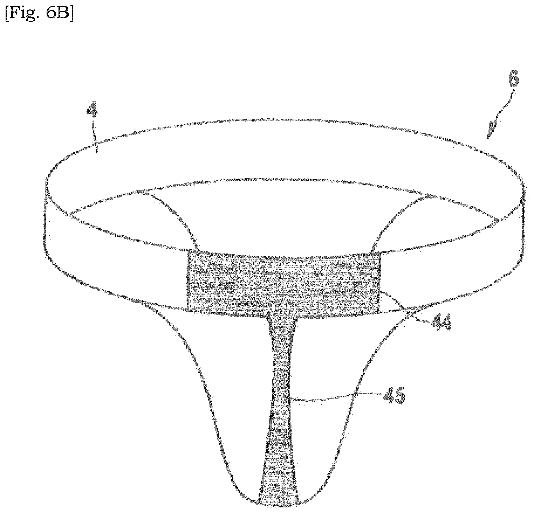

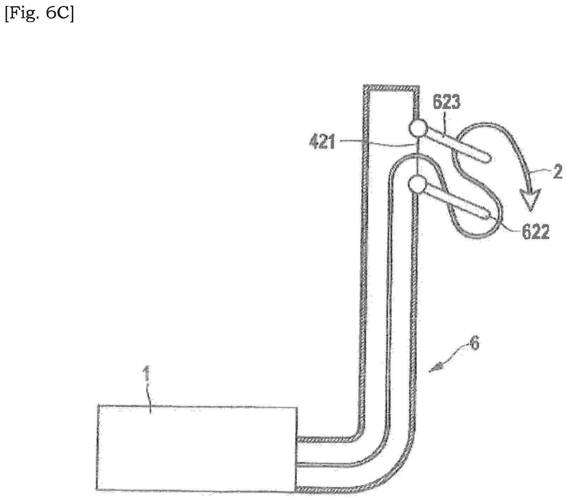

Les figures 6A, 6B, 6C montrent un autre mode de réalisation d'une culotte 6 intégrant le coussin 1 dans la culotte et formant ainsi par combinaison, le dispositif de soutien directionnel externe 100 selon l'invention.

La figure 6A est une vue du devant 61 en perspective de la culotte 6 avec la ceinture 4 correspondant aux modes de réalisation décrits ci-dessus.

Le coussin 1 porte les deux lanières antérieures 2. A l'arrière la culotte 6 a un renfort intégré dans la partie arrière de la culotte et qui est relié à la ceinture 4 et à son renfort 44 et son prolongement 45.

Les deux lanières antérieures 2 passent dans la double paroi avant de la culotte dans deux canaux 611 débouchant sous ou au niveau de la ceinture 4, près des deux anneaux 42a,b. Les lanières antérieures 2 non représentées à la figure 6A sortent pas les deux orifices 421 pour entourer les deux boucles 422, 423 de chaque point de fixation avant 42a,b. (voir aussi Fig. 6C).

La figure 7 montre par une flèche courbe F1, le renvoi vectoriel des forces dans le petit bassin sur le périnée. Elle montre également l'effet du dispositif de maintien 100 pour renvoyer le vecteur force incidente F2 sous la forme d'un vecteur F3 renvoyé, complétant le renvoi vectoriel F1.

De façon plus détaillée, l'effort abdominal entraîne un glissement des organes entre eux (Flèche rouge). Les organes sont élastiques et retenus entre eux par le tissu conjonctif souple (forces de frottement). Ces mouvements retenus par les ligaments (pubo urétraux), provoquent la plicature des tissus les uns sur les autres, à la manière de la plicature du tuyau d'arrosage du jardinier. Cette plicature arrête l'écoulement, on l'appelle zone fonctionnelle de l'urètre.

L'invention permet de réorienter les forces afin que cette plicature puisse se réaliser, grâce à une plaquette étroite rigide et courte gardant sa forme plate, étroite.

Cette plaquette est ajustable contre la partie dure du petit bassin (le coccyx) en arrière et comme indiqué, le dispositif selon l'invention est orientable grâce à l'ajustement de la ceinture de tension avant. En fonction de l'intensité des efforts abdominaux, le renvoi des forces de pression peut être redirigé plus ou moins haut vers l'appui osseux de pubis. Le dispositif bloqué en arrière par l'appui osseux du coccyx, peut ainsi être ajusté et adapté au cours de la journée. La tension réglable des bretelles avant le permet. En effet le dispositif est étroit, et donc mobile entre les autres reliefs osseux latéraux des branches du pubis et de l'Ischion.

Sa position sur l'axe de rotation postérieur est réglable pour réorienter plus ou moins haut les vecteurs des forces réfléchies.

Sa longueur ne dépasse pas les deux tiers postérieurs du périnée, afin de conserver la mobilité indépendante du tiers antérieur. Sa largeur ne dépasse pas la fente vaginale entre les petites lèvres, afin de conserver la mobilité indépendante des parties latérales du périnée.

Cette plaque rigide courte et étroite, ajustable et réglable agit afin de réorienter la direction des frottements dus à la transmission quasi statique des pressions abdominales. Une plicature peut se reproduire, et la zone fonctionnelle et de l'urètre (appelée aussi sphincter) devient active.

### NOMENCLATURE DES ELEMENTS PRINCIPAUX

100 Dispositif de soutien directionnel externe
1 Coussin
11 Plaquette rigide et courte gardant sa forme plate
12 Enveloppe
13 Attache avant
14 Attache arrière
2 Lanières antérieures
3 Lanière postérieure
31 Extrémité de la lanière postérieure
4 Ceinture
41 Fermeture
42a,b Point de fixation avant/double anneau
421 Orifice
422, 423 Anneaux couplés
43 Point de fixation arrière, anneau
44 Renfort
45 Prolongement de renfort arrière
441 Ouverture
5 Culotte combinée à un dispositif de maintien
6 Culotte intégrant le dispositif de maintien

## Revendications

1. Dispositif de soutien directionnel orientable externe (100) de l'urètre féminin à l'effort abdominal, comprenant
- un coussin (1) rectangulaire comprenant une plaquette11 rigide gardant sa forme plate, destiné à être mise en place en avant du coccyx sans couvrir l'ensemble de la surface périnéale et restant à distance de l'orifice urinaire, le coussin (1) étant muni d'une lanière postérieure (3) et de deux lanières antérieures (2)
- la plaquette (11) étant étroite pour rester mobile entre les reliefs osseux latéraux des branches du pubis et les ischions,
- la plaquette étant courte, d'une longueur ne dépassant pas les 2/3 de la longueur du périnée, laissant libre le tiers antérieur du périnée et en étant bloquée à l'arrière par l'appui osseux du coccyx,
- une ceinture abdominale (4) munie d'un point de fixation arrière (43) et de deux points de fixation avant (42a,b) recevant de manière réglable respectivement la lanière postérieure (3) et les deux lanières antérieures (2) pour tenir le coussin rigide (1) contre la zone postérieure et inférieure du périnée en partant du coccyx.

2. Dispositif de soutien directionnel (100) selon la revendication 1,
**caractérisé en ce que**
la ceinture (4) a un renfort postérieur (4) portant le point de fixation arrière (43).

3. Dispositif de soutien directionnel (100) selon la revendication 1,
**caractérisé en ce que**
la ceinture (4) est combinée à une culotte (6).

4. Dispositif de soutien directionnel (100) selon la revendication 3,
**caractérisé en ce que**
le coussin (1) avec la plaquette étroite, rigide, courte gardant sa forme plate est intégré dans la partie intermédiaire (62) de la culotte (6) reliant la partie avant (61) et la partie arrière (63).

5. Dispositif de soutien directionnel (100) selon la revendication 4,
**caractérisé en ce que**
la culotte (6) a une partie arrière (63) renforcée au niveau de la ceinture (4) portant le point de fixation arrière (43) et le fond de culotte présente une partie arrière rigide reliée au renforcement de la liaison interfessiaire de la culotte.

6. Dispositif de soutien directionnel (100) selon la revendication 4,
**caractérisé en ce que**
la culotte (6) a une partie avant (61) munie d'une double paroi formant un canal de passage pour chacune des lanières antérieures pour rejoindre les deux points de fixation réglables.

7. Dispositif de soutien directionnel selon la revendication 2,
**caractérisé en ce que**
la longueur de fixation arrière (43) est réglable.

8. Dispositif de soutien directionnel selon les revendications 4 à 6,
**caractérisé en ce que**
le coussin (1) est intégré dans la région inférieure postérieure de la partie intermédiaire (62) de la culotte (6), l'avant du coussin (1) étant muni de deux lanières antérieures (2) réglables.

## Patentansprüche

1. Äußere verstellbare gerichtete Stützvorrichtung (100) der weiblichen Harnröhre mit abdominaler Einwirkung,
**dadurch gekennzeichnet, dass** sie umfasst
- ein rechteckiges Polster (1), umfassend eine starre Platte 11 zur Beibehaltung seiner flachen Form, dazu bestimmt, vor dem Steißbein angeordnet zu werden, ohne die gesamte perineale Fläche abzudecken, und mit Abstand zur Harnröhrenöffnung, wobei das Polster (1) mit einem hinteren Riemen (3) und zwei vorderen Riemen (2) ausgestattet ist;
- wobei die Platte (11) schmal ist, um zwischen den seitlichen Knochenprofilen des paarigen Schambeins und Sitzbeins beweglich zu bleiben,
- wobei die Platte kurz ist, mit einer Länge, die 2/3 der Länge des Perineums nicht übersteigt, wobei das vordere Drittel des Perineums frei bleibt und sie hinten durch Auflage auf dem Steißbeinknochen arretiert wird,
- einen Bauchgurt (4) mit einem hinteren Befestigungspunkt (43) und zwei vorderen Befestigungspunkten (42a,b), die verstellbar den hinteren Riemen (3) bzw. die zwei vorderen Riemen (2) aufnehmen, um das starre Polster (1) gegen die hintere und untere Zone des Perineums ausgehend vom Steißbein zu halten.

2. Gerichtete Stützvorrichtung (100) nach Anspruch 1,
**dadurch gekennzeichnet, dass**
der Gurt (4) eine hintere Verstärkung (4) aufweist, die den hinteren Befestigungspunkt (43) umfasst.

3. Gerichtete Stützvorrichtung (100) nach Anspruch 1,
**dadurch gekennzeichnet, dass**
der Gurt (4) mit einer Unterhose (6) kombiniert ist.

4. Gerichtete Stützvorrichtung (100) nach Anspruch 3,
**dadurch gekennzeichnet, dass**
- das rechteckige Polster (1) eine starre Platte 11 zur Beibehaltung seiner flachen Form umfasst, dazu bestimmt, vor dem Steißbein angeordnet zu werden, ohne die gesamte perineale Fläche abzudecken, und mit Abstand zur Harnröhrenöffnung, wobei
- die Platte (11) zum einen schmal ist, um zwischen den seitlichen Knochenprofilen des paarigen Schambeins und Sitzbeins beweglich zu bleiben, und zum anderen
- kurz ist, mit einer Länge, die 2/3 der Länge des Perineums nicht übersteigt, wobei das vordere Drittel des Perineums frei bleibt und sie hinten durch Auflage auf dem Steißbeinknochen arretiert wird,
wobei das Polster (1) mit einer schmalen starren kurzen Platte zur Beibehaltung seiner flachen Form in den Zwischenabschnitt (62) der Unterhose (6) integriert ist, der den vorderen Teil (61) und den hinteren Teil (63) verbindet.

5. Gerichtete Stützvorrichtung (100) nach Anspruch 4,
**dadurch gekennzeichnet, dass**
die Unterhose (6) einen auf Höhe des Gurtes (4) verstärkten hinteren Teil (63) aufweist, der den hinteren Befestigungspunkt (43) trägt, und der Schritt der Unterhose einen starren hinteren Teil aufweist, der mit der Verstärkung der zwischen den Gesäßbacken verlaufenden Verbindung der Unterhose verbunden ist.

6. Gerichtete Stützvorrichtung (100) nach Anspruch 4,
**dadurch gekennzeichnet, dass**
die Unterhose (6) einen vorderen Teil (61) mit einer doppelten Lage aufweist, die einen Durchgangskanal für jeden der vorderen Riemen bildet, um die beiden verstellbaren Befestigungspunkte zu erreichen.

7. Gerichtete Stützvorrichtung (100) nach Anspruch 2,
**dadurch gekennzeichnet, dass**
die Länge der hinteren Befestigung (43) verstellbar ist.

8. Gerichtete Stützvorrichtung (100) nach den Ansprüchen 4 bis 6,
**dadurch gekennzeichnet, dass**
das Polster (1) in die hintere untere Region des Zwischenabschnitts (62) der Unterhose (6) integriert ist, wobei die Vorderseite des Polsters (1) mit zwei verstellbaren vorderen Riemen (2) ausgestattet ist.

## Claims

1. External directional support device (100) for the female urethra for abdominal efforts,
**characterised in that** it comprises
- a rectangular cushion (1) comprising a rigid plate (11) which keeps its flat shape, designed to be placed in front of the coccyx without covering the entire perineal area and held at some distance from the urinary orifice, the cushion (1) being provided with a posterior strap (3) and two anterior straps (2).
- the plate (11) is narrow so that it remains mobile between the side bony protrusions of the branches of the pubis and the ischia,
- the plate is short, no longer than 2/3 the length of the perineum, leaving the anterior third of the perineum free and being blocked at the back by the bony support of the coccyx,
- an abdominal belt (4) with a rear attachment point (43) and two front attachment points (42a, b) connected so as to be adjustable regarding the posterior strap (3) and the two anterior straps (2) to hold the rigid cushion (1) against the posterior and lower area of the perineum leading from the coccyx.

2. A directional support device (100) according to claim 1, **characterised**
**in that**
the belt (4) has a posterior reinforcement (4) supporting the rear attaching point (43).

3. A directional support device (100) according to claim 1, **characterised in that** the belt (4) is combined with panties (6).

4. A directional support device (100) according to claim 3, **characterised in that**
- the rectangular cushion (1) comprising a rigid plate 11 which keeps its flat shape, designed to be placed in front of the coccyx without covering the entire perineal area and held at some distance from the urinary orifice of which
- the plate (11) is narrow so that it remains mobile between the side bony protrusions of the branches of the pubis and the ischia, and is
- also short, no longer than 2/3 the length of the perineum, leaving the anterior third of the perineum free while being blocked at the back by
the bony support of the coccyx,
the cushion (1) with a narrow, rigid, and short plate retaining its flat shape is integrated into the intermediate part (62) of the panties (6) connecting the front part (61) and the rear part (63).

5. A directional support device (100) according to claim 4, **characterised**
**in that**
the panties (6) have a rear part (63) reinforced at the belt (4) supporting the rear attachment (43) and the bottom of the panties has a rigid rear part connected to the reinforcement of the intergluteal seam of the panties.

6. A directional support device (100) according to claim 4,
**characterised in that**
the panties (6) have a front part (61) provided with a double wall forming a tubular arrangement through which each of the front straps is fed to join the two adjustable attaching points.

7. A directional support device according to claim 2, **characterised in that**
the length of the rear attachment (43) is adjustable.

8. A directional support device according to claims 4 to 6, **characterised**
**in that**
the cushion (1) is integrated into the lower posterior region of the intermediate part (62) of the panties (6), as the front of the cushion (1) is provided with two adjustable front straps (2).
